# EUROPEAN PATENT APPLICATION

(11) **EP 3 922 983 A2**
(43) Date of publication of application: **15.12.2021**
(21) Application number: 21176878.3
(22) Date of filing: 07.06.2017
(51) Int. Cl.: G01N 21/78, G01N 33/48, G01N 33/52, G01N 33/53, G01N 33/543, G01N 33/558

(54) **IMPROVED LOW SAMPLE VOLUME URINALYSIS ASSAY STRIP, ANALYTICAL KITS, AND METHODS OF USE RELATED THERETO**

(30) Priority: 18.07.2016 US 201662363581 P
(62) Divisional of application: 17831469.6
(71) Applicant: Siemens Healthcare Diagnostics Inc., Tarrytown, NY 10591 (US)
(72) Inventor: LEDDEN, David J., Medwelkhart, Indiana 46514 (US); SCHULMAN, Llyod, Granger , Indiana 46530 (US)
(74) Representative: Schweitzer, Klaus

(57) **Abstract**

Devices and methods for determining the presence or absence of at least one analyte in a liquid test sample.

## Description

### CROSS REFERENCE TO RELATED APPLICATIONS

The present application claims the benefit of and priority to U.S. Provisional Application Serial No. 62/363,581, filed July 18, 2016, the entire disclosure of which is incorporated by reference into the present application.

### STATEMENT REGARDING FEDERALLY FUNDED RESEARCH OR DEVELOPMENT

Not Applicable.

### TECHNICAL FIELD

The presently disclosed and claimed inventive concept(s) relate to a device(s), kit(s), and method(s) for conducting low sample volume urinalysis assays. More specifically, the presently disclosed and claimed inventive concept(s) relate to improved urinalysis assay test trip configurations for the detection of analyte(s) present in low volumes of a patient's urine sample, as well as kits and methods of use related thereto.

### BACKGROUND

Numerous devices and methods exist for detecting analytes that may be present in a patient's fluid sample, including, for instance, a patient's urine sample. Such devices have been proven to be effective in diagnostic assays that detect the presence (or non-presence) as well as the quantity of certain analytes indicative of a patient's health and biological profile, including, but not limited to, analytes and conditions associated with a patient's urine sample. However, these devices, kits, and methods are limited in their configuration in that current configurations do not easily allow for the testing of a small volume of a patient's fluid (i.e., urine) sample. As a result of this configuration, it is difficult to obtain an accurate analysis of a patient's urine sample when a patient only produces a small volume of a liquid test sample (less than about 5 milliliters). In current test strip configurations, when the volume of a sample is low, the receptacle containing the sample must be manipulated (either manually or via machine) to facilitate the interaction between the analyte(s) of interest and the respective reagent(s) contained on the analyte testing pad(s). This can result in inaccurate and/or incomplete results (due to incomplete wetting of the analyte testing regions by the liquid sample), as well as spillage of the sample from the sample receptacle. In addition, current configurations include sizable analyte testing pads along the total (or substantially total) length of the urine test strip (which typically have a length of about 11 centimeters), resulting in the need for increased amounts of reagents to be incorporated on each analyte testing pad. Accordingly, a need exists for new and improved devices, kits, and methods that allow for the detection of at least one analyte of interest which may be present in a low-volume of a patient's liquid test sample. Such devices, kits, and methods thereby allow, by way of example and not by way of limitation, for: (1) the improved detection of the presence (or non-presence) of at least one analyte of interest that may be present in a low-volume of a patient's liquid test sample; (2) the improved detection of the presence (or non-presence) of at least one analyte of interest present in samples of patient populations that produce low-volumes of liquid test sample output (including, but not limited to, newborns, infants, toddlers, young adults, adults, and elderly populations, as well as persons suffering from conditions that restrict urine output, such as dehydration, kidney disease, urethral strictures, and obstructive uropathies); (3) the ability to incorporate smaller and more numerous analyte testing pads on the test strip to thereby increase the number of analytes that can be detected in a low-volume of a patient's liquid test sample; and (4) a reduction in the manufacturing costs associated with the production of such test strips due to a decrease in the amount reagent(s) and materials needed to conduct such diagnostic tests. It is to such devices and methods, as well as kits related thereto, that the presently disclosed and claimed inventive concept(s) is directed.

### DESCRIPTION OF THE SEVERAL VIEWS OF THE DRAWINGS

Figure 1 is a detailed top view of one embodiment of a prior art liquid sample test strip.
Figure 2 is a detailed top view of one embodiment of the improved liquid sample test strip of the presently disclosed and/or claimed inventive concept(s).
Figure 2A is a side view of one embodiment of the improved liquid sample test strip of the presently disclosed and/or claimed inventive concept(s).
Figure 3 is a detailed top view of an alternative embodiment of the improved liquid sample test strip of the presently disclosed and/or claimed inventive concept(s).
Figure 4 is a perspective view of one embodiment of a kit of the presently disclosed and/or claimed inventive concept(s) utilizing the liquid sample test strip depicted in Figures 2 and 2A, as well as an example of one embodiment of a method of use related thereto.

### DETAILED DESCRIPTION

Before explaining at least one embodiment of the inventive concept(s) in detail by way of exemplary drawings, experimentation, results, and laboratory procedures, it is to be understood that the inventive concept(s) is not limited in its application to the details of construction and the arrangement of the components set forth in the following description or illustrated in the drawings, experimentation and/or results. The inventive concept(s) is capable of other embodiments or of being practiced or carried out in various ways. As such, the language used herein is intended to be given the broadest possible scope and meaning; and the embodiments are meant to be exemplary-not exhaustive. Also, it is to be understood that the phraseology and terminology employed herein is for the purpose of description and should not be regarded as limiting.

Unless otherwise defined herein, scientific and technical terms used in connection with the presently disclosed and claimed inventive concept(s) shall have the meanings that are commonly understood by those of ordinary skill in the art. Further, unless otherwise required by context, singular terms shall include pluralities and plural terms shall include the singular. The foregoing techniques and procedures are generally performed according to conventional methods well known in the art and as described in various general and more specific references that are cited and discussed throughout the present specification. The nomenclatures utilized in connection with, and the laboratory procedures and techniques of, analytical chemistry, synthetic organic chemistry, and medicinal and pharmaceutical chemistry described herein are those well-known and commonly used in the art.

All patents, published patent applications, and non-patent publications mentioned in the specification are indicative of the level of skill of those skilled in the art to which this presently disclosed and claimed inventive concept(s) pertains. All patents, published patent applications, and non-patent publications referenced in any portion of this application are herein expressly incorporated by reference in their entirety to the same extent as if each individual patent or publication was specifically and individually indicated to be incorporated by reference.

All of the devices, kits, and/or methods disclosed and claimed herein can be made and executed without undue experimentation in light of the present disclosure. While the compositions and methods of this presently disclosed and claimed inventive concept(s) have been described in terms of preferred embodiments, it will be apparent to those of skill in the art that variations may be applied to the compositions and/or methods and in the steps or in the sequence of steps of the method described herein without departing from the concept, spirit and scope of the presently disclosed and claimed inventive concept(s). All such similar substitutes and modifications apparent to those skilled in the art are deemed to be within the spirit, scope, and concept of the inventive concept(s) as defined by the appended claims.

As utilized in accordance with the present disclosure, the following terms, unless otherwise indicated, shall be understood to have the following meanings:

The use of the word "a" or "an" when used in conjunction with the term "comprising" in the claims and/or the specification may mean "one," but it is also consistent with the meaning of "one or more," "at least one," and "one or more than one." The singular forms "a," "an," and "the" include plural referents unless the context clearly indicates otherwise. Thus, for example, reference to "a compound" may refer to 1 or more, 2 or more, 3 or more, 4 or more or greater numbers of compounds. The term "plurality" refers to "two or more." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects. For example but not by way of limitation, when the term "about" is utilized, the designated value may vary by ± 20% or ± 10%, or ± 5%, or ± 1%, or ± 0.1% from the specified value, as such variations are appropriate to perform the disclosed methods and as understood by persons having ordinary skill in the art. The use of the term "at least one" will be understood to include one as well as any quantity more than one, including but not limited to, 2, 3, 4, 5, 10, 15, 20, 30, 40, 50, 100, etc. The term "at least one" may extend up to 100 or 1000 or more, depending on the term to which it is attached; in addition, the quantities of 100/1000 are not to be considered limiting, as higher limits may also produce satisfactory results. In addition, the use of the term "at least one of X, Y and Z" will be understood to include X alone, Y alone, and Z alone, as well as any combination of X, Y and Z. The use of ordinal number terminology (i.e., "first", "second", "third", "fourth", etc.) is solely for the purpose of differentiating between two or more items and is not meant to imply any sequence or order or importance to one item over another or any order of addition, for example.

As used in this specification and claim(s), the terms "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

As used herein, the term "substantially" means that the subsequently described event or circumstance completely occurs or that the subsequently described event or circumstance occurs to a great extent or degree. For example, the term "substantially" means that the subsequently described event or circumstance occurs at least 90% of the time, or at least 95% of the time, or at least 98% of the time.

As used herein, the phrase "associated with" includes both direct association of two moieties to one another as well as indirect association of two moieties to one another. Non-limiting examples of associations include covalent binding of one moiety to another moiety either by a direct bond or through a spacer group, non-covalent binding of one moiety to another moiety either directly or by means of specific binding pair members bound to the moieties, incorporation of one moiety into another moiety such as by dissolving one moiety in another moiety or by synthesis, and coating one moiety on another moiety.

The term "liquid test sample" as used herein will be understood to include any type of biological fluid sample that may be utilized in accordance with the presently disclosed and claimed inventive concept(s). Examples of biological samples that may be utilized include, but are not limited to, whole blood or any portion thereof (i.e., plasma or serum), saliva, sputum, cerebrospinal fluid (CSF), intestinal fluid, intraperitoneal fluid, cystic fluid, sweat, interstitial fluid, tears, mucus, urine, bladder wash, semen, combinations, and the like. The typical liquid test sample utilized in accordance with the presently disclosed and/or claimed inventive concept(s) is urine. The volume of the sample utilized in accordance with the presently disclosed and claimed inventive concept(s) can be from about 0.1 to about 5 milliliters. As used herein, the term "volume" or "low volume" as it relates to the liquid test sample utilized in accordance with the presently disclosed and claimed inventive concept(s) means from about 0,1 milliliter to about 5 milliliters, or from about 0.5 milliliter to about 4 milliliters, or from about 1 milliliter to about 3 milliliters, or less than or equal to about 2 milliliters.

The term "patient" includes human and veterinary subjects. In certain embodiments, a patient is a mammal. In certain other embodiments, the patient is a human, including, but not limited to, infants, toddlers, children, young adults, adults, and elderly human populations. "Mammal" for purposes of treatment refers to any animal classified as a mammal, including human, domestic and farm animals, nonhuman primates, and zoo, sports, or pet animals, such as dogs, horses, cats, cows, etc.

Turning now to particular embodiments, the presently disclosed and claimed inventive concept(s) relate to a device(s), kit(s), and method(s) for conducting a diagnostic assay(s) for a low volume of a patient's liquid test sample. While a patient's liquid test sample is primarily discussed herein in the context of a patient's urine sample, it should be readily understood by a person having ordinary skill in the art that the presently disclosed and/or claimed inventive concepts have applications to all types of a patient's liquid test sample. More specifically, the presently disclosed and claimed inventive concept(s) relate to an improved urinalysis assay test strip for use in analyte(s) detection assays, as well as kits and methods of use related thereto.

It is contemplated that virtually any reagent used in the fields of biological, chemical, or biochemical analyses and assays could be used in the devices, kits, and methods of the presently claimed and disclosed inventive concept(s). It is contemplated that these reagents may undergo physical and/or chemical changes when bound to an analyte of interest whereby the intensity, color, nature, frequency, wavelength or type of signal generated by the reagent-analyte complex is directly proportional or inversely proportional to the concentration of the analyte existing within the fluid sample. These reagents may contain indicator dyes, metal, enzymes, polymers, antibodies, and electrochemically reactive ingredients and/or chemicals that, when reacting with an analyte(s) of interest, may exhibit a change in color, fluorescence, or wavelength.

Any method of detecting and measuring the analyte in a fluid sample can be used in the devices, kits, and methods of the presently claimed and inventive concepts, including, but not limited to, visual inspection of a detectable color change due to the association between the analyte and reagent. A variety of assays for detecting analytes are well known in the art and include, but are not limited to, chemical assays, enzyme inhibition assays, antibody stains, latex agglutination, latex agglutination inhibition and immunoassays, such as, radioimmunoassays.

Assays, including, but not limited to, immunoassays, chemical and/or chemical-based assays, and nucleic acid capture assays can be developed for a multiplexed panel of proteins, peptides, and nucleic acids which may be contained within a liquid test sample, with such proteins, peptides, and compounds including, for example but not by way of limitation, albumin, microalbumin, cholesterol, triglycerides, high-density lipoproteins, low-density lipoproteins, hemoglobin, myoglobin, α-1-microglobin, immunoglobins, enzymes, proteins, glycoproteins, protease inhibitors, drugs, cytokines, albumin, creatinine, bilirubin, ketones (including, but not limited to, acetoacetic acid), urobilinogen, nitrites, leukocytes (including, but not limited to, leukocyte esterase), blood, and glucose. In addition, the presently disclosed and/or claimed inventive concept(s) can detect certain conditions associated with a patient's liquid test sample, including, but not limited to, a sample's specific gravity and/or pH. As disclosed herein, the device(s), kit(s), and method(s) disclosed and/or claimed herein may be used for the analysis of any fluid sample, including, without limitation, whole blood, plasma, serum, or, preferably, urine. Typical instances related to the currently disclosed and/or claimed inventive concept(s) involve urine as the liquid test sample.

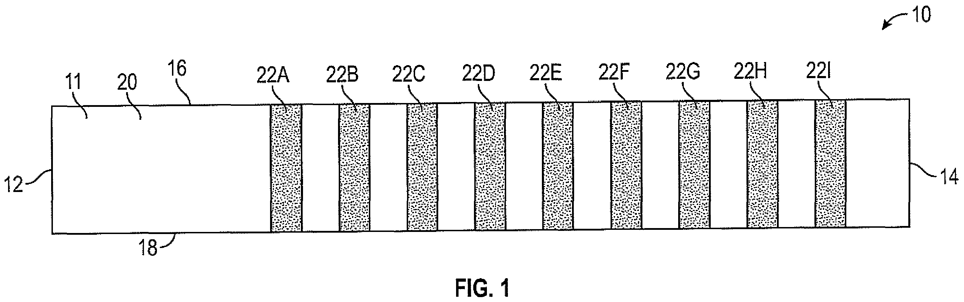

Referring now to the Figures, and more particularly to FIG. 1, shown therein is an embodiment of a prior art liquid sample test strip 10. The test strip 10 comprises a substrate 11 having a first end 12, a second end 14, a first side 16, a second side 18, a top surface 20, a bottom surface (not shown), and a plurality of analyte testing regions 22A-22I. The plurality of analyte testing regions 22A-22I extend linearly on the top surface 20 of the substrate 11 along the substantially entire length of the substrate 11 from the first end 12 to the second end 14. Each of the analyte testing regions 22A-22I contain a reagent (not shown) which produces a predictable color change on such analyte testing regions 22A-22I when exposed to specific analytes which may be present in a liquid test sample. Such analyte testing regions 22A-22I are then visually interrogated (either manually or by an optical device) to determine the specific analyte's(s') level(s) and/or concentration(s). In order to obtain analyte readings from the test strip 10, one of two methods are typically employed by a user: (1) the test strip 10 is disposed within a receptacle (not shown) which contains the liquid test sample such that the analyte testing regions 22A-22I are submerged in the liquid test sample to allow for association with the reagent contained on each of the analyte testing regions 22A-22I and the particular analytes of interest which may be contained in the liquid test sample; or (2) the user individually pipettes the liquid test sample contained in the receptacle on each of the individual analyte testing regions 22A-22I. Each of the above testing methods suffer from a number of limitations-limitations which are remedied by the presently disclosed and/or claimed inventive concepts. First, with respect to method (1), when there is only a small volume (less than about 5 milliliters) of the liquid test sample, a user (or machine) must tip the receptacle on its side and/or rotate the receptacle containing the liquid test sample to ensure that each of the analyte testing regions 22A-22I is adequately wetted by the liquid test sample. The tipping and/or rotation of the receptacle can result in spillage of the liquid test sample from the receptacle, thereby resulting in loss of liquid test sample volume and/or contamination of the liquid test sample, and/or potentially increasing the risk for a biohazard situation. In addition, the manual dipping of the test strip 10 into a receptacle by a user can result in the deformation of the test strip 10, thereby decreasing the functionality of the test strip 10 due to such deformation. Second, with respect to method (2), while dosing the individual analyte testing regions 22A-22I ensures adequate coverage of the analyte testing regions 22A-221 with the liquid test sample, such dosing is time consuming and unnecessarily labor intensive.

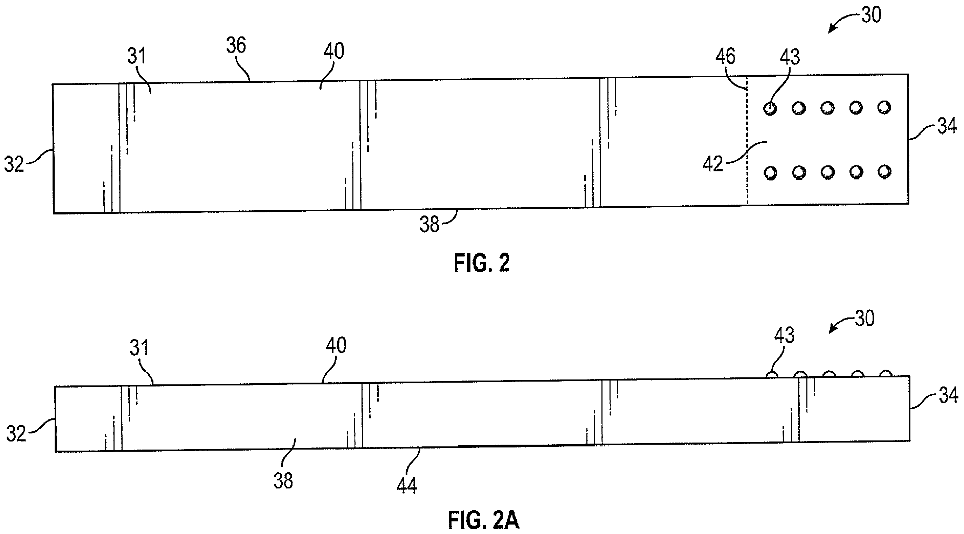

Referring now to FIGS. 2 and 2A, shown therein is an embodiment of an improved liquid sample test strip 30 constructed in accordance with the presently disclosed and/or claimed inventive concept(s). The liquid sample test strip 30 comprises a substrate 31 and an analyte testing region 42 which contains a plurality of analyte testing pads 43. The liquid sample test strip 30 has typical dimensions (as measured in length by width) of about 9 centimeters to about 11 centimeters by about 0.5 centimeter to about 1 centimeter; however, a person having ordinary skill in the art should appreciate that the dimensions of the liquid sample test strip 30 can be of any length and width that is suitable for accomplishing the presently disclosed and/or claimed inventive concept(s). In typical embodiments of the presently disclosed and/or claimed inventive concept(s), the length of the liquid sample test strip 30 is greater than its width.

The substrate 31 comprises a first end 32, a second end 34, a first side 36, a second side 38, a top surface 40, and a bottom surface 44. In typical embodiments of the presently disclosed and/or claimed inventive concept(s), the first side 36 and the second side 38 comprise a length that is substantially longer than the first end 32 and the second end 34. By way of example and not by way of limitation, the length of the first side 36 and the second side 38 may be as high as 20 times, 15 times, 10 times, 9 times, 8 times, 7 times, 6 times, 5 times, 4 times, 3 times, or 2 times the length of the first end 32 and the second end 34. While FIGS. 2-2A depict the substrate 31 as being substantially rectangular in shape, it should be understood to a person having ordinary skill in the art that the substrate 31 can be any shape that allows for the liquid sample test strip 30 to be substantially introduced and disposed within a receptacle containing a liquid test sample such that the analyte testing region 42 is submerged (or, adequately wetted) by the liquid test sample. The substrate 31 can be constructed of any material that accomplishes the presently disclosed and/or claimed inventive concept(s), including, but not limited to, nitrocellulose, cellulose acetate, Mylar^{®}, polyester film, polyethylene terephthalate, polycarbonate, polystyrene, or combinations thereof.

In one embodiment, the analyte testing region 42 is located on the top surface 40 of the substrate 31 and extends from the second end 34 along a first axis substantially parallel to the first side 36 and second side 38 of the substrate 31 to a second axis 46 substantially parallel to the second side 38. The analyte testing region 42 may be formed of the same material as the substrate 31, or may be formed from a different material, including, but not limited to, nitrocellulose. The distance from the second end 34 to the second axis 46 is typically about 0.5 centimeters to about 5 centimeters, or from about 1 centimeter to about 4 centimeters, or from about 1 centimeter to about 3 centimeters, or from about 2 centimeters to about 3 centimeters, or less than or equal to about 3 centimeters, or any distance by which the analyte testing region 42 is submerged (or, adequately wetted) by the liquid test sample when disposed within the liquid test sample receptacle. Although shown in FIGS. 2-2A as being located on the top surface 40 of the substrate 31, the analyte testing region 42 may be located on the bottom surface 44 of the substrate 31. In another embodiment, there may be an analyte testing region 42 located on both the top surface 40 and the bottom surface 42 of the substrate 31.

The analyte testing region 42 comprises a plurality of analyte testing pads 43. The plurality of analyte testing pads 43 comprise at least one reagent (not shown) that is capable of associating with at least one analyte when such at least one analyte is present in the liquid test sample. The plurality of analyte testing pads 43 may be formed on the substrate 31 via any method that accomplishes the presently disclosed and/or claimed inventive concept(s). By way of example, and not by way of limitation, the plurality of analyte testing pads 43 may be formed by (either during the liquid sample test strip 30 manufacturing process or via an individual user post-manufacturing) spotting a dot of the at least one reagent within the analyte testing region 42. Alternatively, or in addition to, a strip (or multiple strips) containing at least one reagent (or, the at least one reagent may be disposed thereon later) can be adhered to the substrate 31 within the analyte testing region 42 and the strip(s) can be cut (either manually or via machine) to form the plurality of analyte testing pads 43. Additionally, by way of example only, the analyte testing pads 43 may be constructed of a separate material, for instance, absorbent pads and/or micro-punches of reaction paper(s) that are adhered (for example, via non-reactive glue(s)) within the analyte reagent region 42 on the substrate 31. Such separate material and/or reaction paper(s) can be any material commonly known in the art and/or which is capable of accomplishing the presently disclosed and/or claimed inventive concept(s). Accordingly, in certain embodiments, the at least one reagent can itself/themselves form the plurality of analyte testing pads 43 on the substrate 31 or such at least one reagent can be disposed on a separately constructed material that is adhered to the substrate 31 to thereby form the plurality of analyte testing pads 43.

While FIGS. 2-2A depict ten circular analyte testing pads 43 arranged in an array comprising two columns and five rows (in which the columns are oriented substantially parallel to the first side 36 and the second side 38 of the substrate 31 and the rows are oriented substantially parallel to the first end 32 and the second end 34 of the substrate 31) within the analyte testing region 42, it should be understood to a person having ordinary skill in the art that the number, shape, and orientation of the analyte testing pads 43 can be varied to accomplish the presently disclosed and/or claimed inventive concept(s). For example, the plurality of analyte testing pads 43 can comprise 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, or 100 analyte testing pads 43, provided that such plurality of analyte testing pads 43 are contained within the analyte testing region 42. In one embodiment, the analyte testing region 42 comprises at least three analyte testing pads 43. Similarly, the plurality of analyte testing pads 43 can be configured to be any shape capable of containing at least one reagent, including, but not limited to, circular, triangular, square, rectangular, pentagonal, hexagonal, heptagonal, octagonal, or combinations thereof.

As previously mentioned, while FIGS. 2-2A show the orientation of the plurality of analyte testing pads 43 being arranged in an array of two columns and five rows, a person having ordinary skill in the art should appreciate that the plurality of analyte testing pads 43 can be arranged in any orientation and/or configuration that accomplishes the presently disclosed and/or claimed inventive concept(s). By way of example, and not by way of limitation, in one embodiment the configuration of the plurality of analyte testing pads 43 within the analyte testing region 42 may be oriented in one row, at least one row, or at least one row and at least two or more columns. In one embodiment, the plurality of analyte testing pads 43 are oriented in one row, in which the one row is oriented substantially parallel to the first end 32 and second end 34 of the substrate 31.

In another embodiment, the plurality of analyte testing pads 43 are oriented in one row and at least two columns, in which the one row is oriented substantially parallel to the first end 32 and the second end 34 of the substrate 31 and the two or more columns are oriented substantially parallel to the first side 36 and the second side 38 of the substrate 31. In addition, the orientation of the plurality of analyte testing pads 43 need not be uniform in its configuration. For example, each row need not comprise two analyte testing pads 43 and each column need not comprise five analyte testing pads 43-each row and column may contain any number of analyte testing pads 43 that accomplishes the presently disclosed and/or claimed inventive concept(s). Likewise, the plurality of analyte testing pads 43 may be configured in a random orientation, provided that the plurality of analyte testing pads 43 are contained within the analyte testing region 42. In another embodiment, each analyte testing pad 43 is in (or is itself) a column and/or a row-for instance, by way of example, and not by way of limitation, each of the analyte testing pads 43 may be oriented within the analyte testing region 42 along an axis substantially parallel to the second end 34 of the substrate 31. In another embodiment, the analyte testing pads 43 are oriented along an axis substantially parallel to the second end 34 of the substrate 31 substantially near the second end 34 of the substrate 31 within the analyte testing region 42. In such a configuration, the analyte testing pads 43 may be oriented in one or more rows and multiple columns within the analyte testing region 42, wherein each row comprises one or more analyte testing pads 43 and each column comprises one or more analyte testing pads 43. In addition, each of the analyte testing pads 43 may be oriented in at least two columns within the analyte testing region 42, the two columns being situated along an axis substantially parallel to the first side 36 and second side 38 of the substrate 31.

Each of the analyte testing pads 43 may contain one or more of the same, different, or combinations of same and different reagents. In one embodiment, an analyte testing region 42 may contain two or more analyte testing pads 43 containing the same reagent, which allows for the redundant detection of the same analyte, thereby providing replicate detection of the particular analyte which facilitates greater accuracy, precision, and confidence in such detection. In another embodiment, the plurality of analyte testing pads 43 comprise at least two duplicate analyte testing pads 43 for a first analyte being tested, wherein each of the at least two duplicate analyte testing pads 43 contain the same reagent. The at least two duplicate analyte testing pads 43 for the first analyte being tested may be clustered together within the analyte testing region 42 (for example, the duplicate analyte testing pads 43 may be adjacent to one other in same column and/or row). Alternatively, the at least two duplicate analyte testing pads 43 for the first analyte being tested may be spaced and oriented apart from one another within the analyte testing region 42. Furthermore, the analyte testing region 42 can contain extra, similar duplicate analyte resting pads 43 for analytes in addition to the duplicate analyte resting pads 43 for the first analyte (e.g., a second analyte, a third analyte, etc...).

In another embodiment, a plurality of analyte testing pads 43 are oriented in a first array within the analyte testing region 42, wherein the plurality of analyte testing pads 43 of the first array contain reagent(s) corresponding to the particular analytes being tested. This latter embodiment may additionally comprise a plurality of analyte testing pads 43 oriented in a second duplicate array, wherein the plurality of analyte testing pads 43 of the second duplicate array contain at least the same reagent(s) of the plurality of analyte testing pads 43 of the first array to provide the capacity for redundant and/or replicate detection of the particular analytes being tested. The second duplicate array may be contained within the analyte testing region 42 (for, by way of example, redundant testing of low volumes of liquid samples) or outside the analyte testing region 42 (for, by way of example, redundant testing of the liquid sample as the volume of the liquid sample permits). This latter embodiment is not limited to only a first and second array and may include any number of arrays comprising a plurality of analyte testing pads 43 that accomplishes the presently disclosed and/or claimed inventive concept(s). Futhermore, the second duplicate array may contain all or less than all of the analyte testing pads 43 contained in the first array. In an example, the second duplicate array can be spaced further from the second end 34 as compared to the first array.

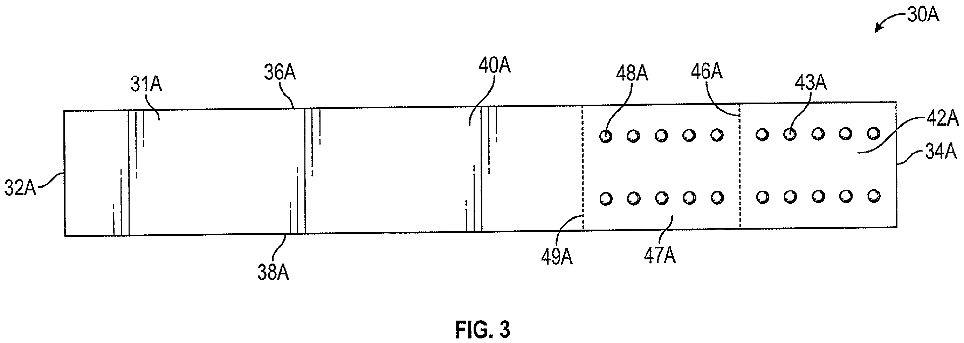

Referring now to FIG. 3, shown therein is an alternative embodiment of a liquid sample test strip 30A. Liquid sample test strip 30A is substantially similar to liquid sample test strip 30 (as depicted in FIGS. 2-2A), with the exception that the liquid sample test strip 30A further comprises a calibration region 47A having a plurality of calibration pads 48A. The inclusion of the calibration region 47A is facilitated by the small area footprint of the analyte testing region 42A and the calibration region 47A, as both are located substantially near the second end 34A of the substrate 31A. The addition of the plurality of calibration pads 48A allows for lot specific calibration of the liquid sample test strip 30A, thereby increasing the accuracy and precision of the detection of at least one analyte that may be present in a liquid test sample.

In one embodiment, the calibration region 47A is located on the top surface 40A of the substrate 31A and extends from a second axis 46A along an axis substantially parallel to the first side 36A and second side 38A of the substrate 31A to a third axis 49A that is substantially parallel to the second side 38A and the second axis 46A. The calibration region 47A may be formed of the same material as the substrate 31A, or may be formed from a different material, including, but not limited to, nitrocellulose. The distance from the second axis 46A to the third axis 46 is typically about 0.5 centimeters to about 3 centimeters, or from about 1 centimeter to about 2 centimeters, or from about 1 centimeter to about 1.5, or less than or equal to about 1.5 centimeters, or any distance by which the calibration region 47A and the analyte testing region 42A are submerged (or, adequately wetted) by the liquid test sample when disposed within the liquid test sample receptacle. Although shown in FIG. 3 as being located on the top surface 40A of the substrate 31A, the calibration region 47A may be located on the bottom surface (not shown) of the substrate 31A. In another embodiment, a calibration region 47A is located on both the top surface 40A and the bottom surface (not shown) of the substrate 31A.

The calibration region 47A comprises a plurality of calibration pads 48A. The plurality of calibration pads 48A comprise at least one reagent (not shown) that is capable of associating with at least one analyte when such at least one analyte is present in the liquid test sample. The plurality of calibration pads 48A may be formed on the substrate 31A via any method that accomplishes the presently disclosed and/or claimed inventive concept(s). By way of example, and not by way of limitation, the plurality of calibration pads 43 may be formed by (either during the liquid sample test strip 30A manufacturing process or via an individual user post-manufacturing) spotting a dot of the at least one reagent within the calibration region 47A. Alternatively, or in addition to, a strip (or multiple strips) containing at least one reagent (or, the at least one reagent may be disposed thereon later) can be adhered to the substrate 31A within the calibration region 47A and the strip(s) can be cut (either manually or via machine) to form the plurality of calibration pads 48A. Additionally, by way of example only, the calibration pads 48A may be constructed of a separate material, for instance, absorbent pads and/or micro-punches of reaction paper(s) that are adhered (for example, via non-reactive glue(s)) within the calibration region 47A on the substrate 31A. Such separate material and/or reaction paper(s) can be any material commonly known in the art and/or which is capable of accomplishing the presently disclosed and/or claimed inventive concept(s). Accordingly, in certain embodiments, the at least one reagent can itself/themselves form the plurality of calibration pads 48A on the substrate 31A, or such at least one reagent can be disposed on a separately constructed material that is adhered to the substrate 31A to thereby form the plurality of calibration pads 48A.

While FIG. 3 depicts ten circular calibration pads 48A arranged in an array comprising two columns and five rows within the calibration region 47A, it should be understood to a person having ordinary skill in the artthatthe number, shape, and orientation of the calibration pads 48A can be varied to accomplish the presently disclosed and/or claimed inventive concept(s). For example, the plurality of calibration pads 48A can comprise 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, or 100 calibration pads 48A, provided that such plurality of calibration pads 48A are contained within the calibration region 47A. In one embodiment, the calibration region 47A comprises at least three calibration pads 48A. Similarly, the plurality of calibration pads 48A can be configured to be any shape capable of containing at least one reagent, including, but not limited to, circular, triangular, square, rectangular, pentagonal, hexagonal, heptagonal, octagonal, or combinations thereof. As previously mentioned, while FIG. 3 shows the orientation of the plurality of calibration pads 48A being arranged in an array of two columns and five rows, a person having ordinary skill in the art should appreciate that the plurality of analyte calibration pads 48A can be arranged in any orientation and/or configuration that accomplishes the presently disclosed and/or claimed inventive concepts. By way of example, and not by way of limitation, in one embodiment the configuration of the plurality of calibration pads 48A within the calibration region 47A may be oriented in one row and two or more columns, in which the one row is oriented substantially parallel to the first end 32A and the second end 34A of the substrate 31A and the two or more columns are oriented substantially parallel to the first side 36A and the second side 38A of the substrate 31A. In addition, the orientation of the plurality of calibration pads 47A need not be uniform in its configuration. For example, each row need not comprise two calibration pads 48A and each column need not comprise five calibration pads 48A-each row and column may contain any number of calibration pads 48A that accomplishes the presently disclosed and/or claimed inventive concept(s). Likewise, the plurality of calibration pads 48A may be configured in a random orientation, provided that the plurality of calibration pads 48A are contained within the calibration region 47A. Each of the calibration pads 48A may contain one or more of the same, different, or combinations of same and different reagents.

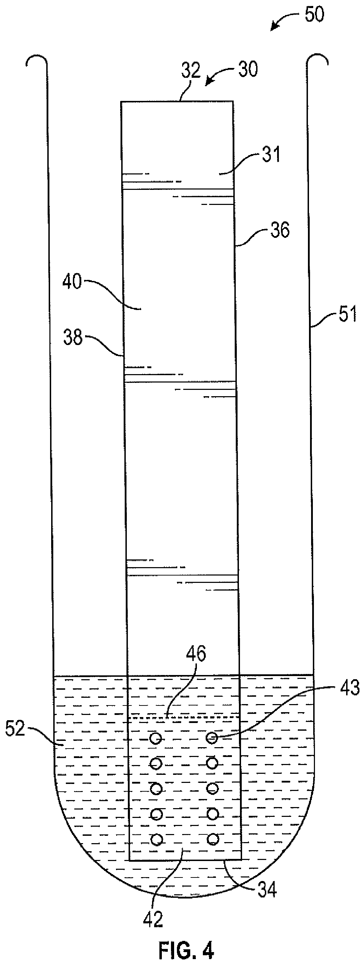

Referring now to FIG. 4, shown therein is an illustrative, non-limiting embodiment of a kit 50 (and method of using such kit) constructed and used in accordance with the presently disclosed and/or claimed inventive concept(s). In one embodiment, the kit 50 comprises the liquid sample test strip 30 as shown in FIGS. 2-2A and a receptacle 51. However, the liquid sample test strip depicted in FIG. 3 is also well suited for use in the kit 50. The liquid sample test strip 30 (and liquid sample test strip 30A) structure has previously been described herein. The receptacle 51 is configured to receive and contain a liquid test sample 52. In one embodiment, the receptacle 51 comprises a test tube, a cuvette, or a flask, all of which are commonly known in the art while the liquid test sample 52 comprises a patient's urine having a volume from about 0.1 milliliter to about 5 milliliters, or from about 0.5 milliliter to about 4 milliliters, or from about 1 milliliter to about 3 milliliters, or less than or equal to about 2 milliliters. A person having ordinary skill in the art, however, should appreciate that the receptacle 51 can be any item configured to receive and contain the liquid test sample 51 and the liquid sample test strip 30. Additionally, the receptacle is configured to receive the liquid sample test strip 30, such that the analyte testing region 42 comprising the plurality of analyte testing pads 43 is submerged (or, adequately wetted) within (or by) the liquid test sample 52 contained within the receptacle 51. Upon introduction of the liquid sample test strip 30 into the receptacle 51, the at least one reagent contained on (or, alternatively, forming) the plurality of analyte testing pads 43 associate with at least one analyte within the liquid test sample 52 when at one analyte is present within the liquid test sample 50. When association occurs between the at least one reagent on (or, alternatively, forming) the plurality of analyte testing pads 43, the plurality of analyte testing pads 43 (due to the association between the at least one reagent and the at least one analyte) produce a detectable a response when the at least one analyte is present in the liquid test sample 52. The detectable response can be qualitative (for example, a change in color) which can be measured manually by a user (for instance, by visual inspection) or via a machine, or may be semi-quantitative or quantitative (for example, a measure of the concentration of the particular analyte present within the liquid test sample 52). Accordingly, when at least one analyte is present in the liquid test sample 52, a color change occurs to the plurality analyte testing pads 43 thereby allowing a user (or machine) to detect and measure the presence of the at least one analyte present in the test sample 52.

### NON-LIMITING EXAMPLES OF THE INVENTIVE CONCEPT(S)

A test strip device for detecting at least one analyte in a liquid test sample, comprising: a substrate, the substrate comprising a first end, a second end, a first side, a second side, a top surface, and a bottom surface, wherein the first side and the second side comprise a length that is substantially longer than the first end and the second end; and an analyte testing region, wherein the analyte testing region is located on the top surface of the substrate and extends from the second end of the substrate along a first axis substantially parallel to the first side and the second side of the substrate to a second axis substantially parallel to the second end of the substrate, and further wherein the analyte testing region comprises at least two columns of analyte testing pads, wherein the at least two columns are substantially parallel to the first axis and each of the at least two columns comprises at least one analyte testing pad, the analyte testing pads comprising at least one reagent capable of associating with at least one analyte present in a liquid test sample to produce a detectable response in the presence of the at least one analyte.

The test strip device of, wherein the at least two columns comprise at least ten analyte testing pads, and further wherein the at least ten analyte testing pads are arranged in an array comprising at least two rows and at least two columns.

The test strip device, wherein the analyte testing pads comprise the same, different, or a combination of same and different reagents.

The test strip device, wherein the substrate is selected from the group consisting of nitrocellulose, cellulose acetate, Mylar^{®}, polyester film, polyethylene terephthalate, polycarbonate, and polystyrene, or combinations thereof.

The test strip device, wherein the liquid test sample is urine, and further wherein the liquid test sample comprises a volume of about 0.1 milliliters to about 3 milliliters.

The test strip device, wherein the at least one analyte is selected from the group consisting of glucose, bilirubin, ketones, blood, proteins, urobilinogen, nitrites, leukocytes, albumin, creatinine, ascorbic acid, specific gravity, and pH.

The test strip device, wherein the at least one reagent is selected from the group consisting of glucose oxidase, peroxidase, potassium iodide, 2,4-dichloroaniline diazonium salt, sodium nitroprusside, bromthymol blue, methyl vinyl ether, maleic anhydride, sodium hydroxide, diispropylbenzene dihydroperoxide, 3, 3', 5, 5'-tetramethylbenzidine, methyl red, tetrabromphenol blue, p-diethylamino-benzaldehyde, p-arsanilic acid, 1, 2, 3, 4-tetrahydrobenzo(h) quinolin-3-ol, derivatized pyrrole amino acid ester, bis (3',3"-diiodo-4',4"-dihydroxy-5',5"-dinitrophenyl)-3,4,5,6-tetrabromosulfonepthalein, copper sulfate, and diazonium salt, or combinations thereof.

The test strip device, wherein the detectable response comprises a color change to the at least two analyte testing pads.

A test strip device for detecting at least one analyte present in a liquid test sample, comprising: a substrate, the substrate comprising a first end, a second end, a first side, a second side, a top surface, and a bottom surface, wherein the first side and the second side comprise a length that is substantially longer than the first end and the second end; and an analyte testing region, wherein the analyte testing region is located on the top surface of the substrate and extends from the second end of the substrate along a first axis substantially parallel to the first side and the second side of the substrate to a second axis substantially parallel to the second end of the substrate, further wherein the second axis is about 0.5 centimeter to about 3 centimeters from the second end of the substrate, and further wherein the analyte testing region comprises at least ten analyte testing pads, the analyte testing pads comprising at least one reagent capable of associating with at least one analyte present in a liquid test sample to produce a detectable response in the presence of the at least one analyte, wherein the analyte testing pads are oriented in at least one row within the analyte testing region, the at least one row being substantially parallel to the first end and the second end of the substrate.

The test strip device, wherein the at least ten analyte testing pads are arranged in an array within the analyte testing region, the array comprising at least two rows and at least two columns, wherein the at least two rows are substantially parallel to the first end and the second end of the substrate, and further wherein the at least two columns are substantially parallel to the first side and the second side of the substrate.

The test strip device, wherein the at least ten analyte testing pads comprise the same, different, or a combination of same and different reagents.

The test strip device, wherein the liquid test sample is urine, and further wherein the liquid test sample comprises a volume of about 0.1 milliliters to about 3 milliliters.

The test strip device, wherein the at least one analyte is selected from the group consisting of glucose, bilirubin, ketones, blood, proteins, urobilinogen, nitrites, leukocytes, albumin, creatinine, ascorbic acid, specific gravity, pH, and combinations thereof.

The test strip device, wherein the at least one reagent is selected from the group consisting of glucose oxidase, peroxidase, potassium iodide, 2,4-dichloroaniline diazonium salt, sodium nitroprusside, bromthymol blue, methyl vinyl ether, maleic anhydride, sodium hydroxide, diispropylbenzene dihydroperoxide, 3, 3', 5, 5'-tetramethylbenzidine, methyl red, tetrabromphenol blue, p-diethylamino-benzaldehyde, p-arsanilic acid, 1, 2, 3, 4-tetrahydrobenzo(h) quinolin-3-ol, derivatized pyrrole amino acid ester, bis (3',3"-diiodo-4',4"-dihydroxy-5',5"-dinitrophenyl)-3,4,5,6-tetrabromosulfonepthalein, copper sulfate, and diazonium salt, or combinations thereof.

A method for performing analytical reactions to determine the presence or absence of an analyte in a liquid test sample, the method comprising the steps of: obtaining a liquid test sample from a patient and disposing the liquid test sample in a receptacle; introducing a test strip device into the receptacle, the test strip device comprising: a substrate, the substrate comprising a first end, a second end, a first side, a second side, a top surface, and a bottom surface, wherein the first side and the second side comprise a length that is substantially longer than the first end and the second end; and an analyte testing region, wherein the analyte testing region is located on the top surface of the substrate and extends from the second end of the substrate along a first axis substantially parallel to the first side and the second side of the substrate to a second axis substantially parallel to the second end of the substrate, further wherein the second axis is about 1 centimeter to about 3 centimeters from the second end of the substrate, and further wherein the analyte testing region comprises at least two analyte testing pads, the analyte testing pads comprising at least one reagent capable of associating with at least one analyte present in the liquid test sample contained with the receptacle to produce a detectable response in the presence of the at least one analyte; measuring the detectable response produced to determine the presence or absence of at least one analyte present in the liquid test sample.

The method, wherein the detectable response comprises a color change to the at least two analyte testing pads.

An analytical reaction kit, the kit comprising: a test strip device, the test strip comprising: a substrate, the substrate comprising: a first end, a second end, a first side, a second side, a top surface, and a bottom surface; and an analyte testing region, wherein the analyte testing region is located on the top surface of the substrate and extends from the second end of the substrate along a first axis substantially parallel to the first side and the second side of the substrate to a second axis substantially parallel to the second end of the substrate, further wherein the second axis is about 1 centimeter to about 3 centimeters from the second end of the substrate, and further wherein the analyte testing region comprises at least two analyte testing pads, the analyte testing pads comprising at least one reagent capable of associating with at least one analyte present in a liquid test sample to produce a detectable response in the presence of the at least one analyte; and a receptacle, wherein the receptacle is configured to receive and contain the liquid test sample and the test strip device such that the analyte testing pads are submerged in the liquid test sample when the test strip device is disposed within the receptacle.

The analytical reaction kit, wherein the receptacle is selected from the group consisting of a test tube, a cup, and a flask.

The analytical reaction kit, wherein the substrate is selected from the group consisting of nitrocellulose, cellulose acetate, Mylar^{®}, polyester film, polyethylene terephthalate, polycarbonate, and polystyrene.

The analytical reaction kit, wherein the liquid test sample is urine, and further wherein the liquid test sample comprises a volume of about 0.1 milliliters to about 3 milliliters.

The analytical reaction kit, wherein the at least one analyte is selected from the group consisting of glucose, bilirubin, ketones, blood, proteins, urobilinogen, nitrites, leukocytes, albumin, creatinine, ascorbic acid, specific gravity, and pH.

The analytical reaction kit, wherein the at least one reagent is selected from the group consisting of glucose oxidase, peroxidase, potassium iodide, 2,4-dichloroaniline diazonium salt, sodium nitroprusside, bromthymol blue, methyl vinyl ether, maleic anhydride, sodium hydroxide, diispropylbenzene dihydroperoxide, 3, 3', 5, 5'-tetramethylbenzidine, methyl red, tetrabromphenol blue, p-diethylamino-benzaldehyde, p-arsanilic acid, 1, 2, 3, 4-tetrahydrobenzo(h) quinolin-3-ol, derivatized pyrrole amino acid ester, bis (3',3"-diiodo-4',4"-dihydroxy-5',5"-dinitrophenyl)-3,4,5,6-tetrabromosulfonepthalein, copper sulfate, and diazonium salt.

The analytical reaction kit, wherein the detectable response comprises a color change to the at least two analyte testing pads.

The analytical reaction kit, wherein the device further comprises at least ten analyte testing pads.

Thus, in accordance with the presently disclosed and claimed inventive concept(s), there have been provided devices, kits, and methods for detecting at least one analyte present in a patient's low-volume liquid test sample. As described herein, the presently disclosed and claimed inventive concept(s) relate to embodiments of improved low-sample volume urinalysis assay strips for use in analyte(s) detection assay, as well as kits and methods of use related thereto. Such presently disclosed and/or claimed inventive concept(s) fully satisfy the objectives and advantages set forth hereinabove. Although the presently disclosed and claimed inventive concept(s) has been described in conjunction with the specific drawings, experimentation, results and language set forth hereinabove, it is evident that many alternatives, modifications, and variations will be apparent to those skilled in the art. Accordingly, it is intended to embrace all such alternatives, modifications and variations that fall within the spirit and broad scope of the presently disclosed and claimed inventive concept(s).

## Claims

1. A test strip device for detecting at least one analyte present in a liquid test sample, comprising:
a substrate, the substrate comprising a first end, a second end, a first side, a second side, a top surface, and a bottom surface, wherein the first side and the second side comprise a length that is substantially longer than the first end and the second end; and
an analyte testing region, wherein the analyte testing region is located on the top surface of the substrate and extends from the second end of the substrate along a first axis substantially parallel to the first side and the second side of the substrate to a second axis substantially parallel to the second end of the substrate, further wherein the second axis is about 0.5 centimeter to about 3 centimeters from the second end of the substrate, and further wherein the analyte testing region comprises at least ten analyte testing pads, the analyte testing pads comprising at least one reagent capable of associating with at least one analyte present in a liquid test sample to produce a detectable response in the presence of the at least one analyte,
wherein the at least ten analyte testing pads comprise different reagents or a combination of same and different reagents,
wherein the analyte testing pads are oriented in at least one row within the analyte testing region, the at least one row being substantially parallel to the first end and the second end of the substrate.

2. The device of claim 1, wherein the at least ten analyte testing pads are arranged in an array within the analyte testing region, the array comprising at least two rows and at least two columns, wherein the at least two rows are substantially parallel to the first end and the second end of the substrate, and further wherein the at least two columns are substantially parallel to the first side and the second side of the substrate.

3. The device of claim 1, wherein the substrate is selected from the group consisting of nitrocellulose, cellulose acetate, Mylar^{®}, polyester film, polyethylene terephthalate, polycarbonate, and polystyrene, or combinations thereof.

4. The device of claim 1, wherein the detectable response comprises a color change to the at least two analyte testing pads.

5. The device of claim 1, wherein the liquid test sample is urine.

6. The device of claim 5, wherein the liquid test sample comprises a volume of about 0.1 milliliters to about 3 milliliters.

7. The device of claim 1, wherein the at least one analyte is selected from the group consisting of glucose, bilirubin, ketones, blood, proteins, urobilinogen, nitrites, leukocytes, albumin, creatinine, ascorbic acid, specific gravity, pH, and combinations thereof.

8. The device of claim 1, wherein the at least one reagent is selected from the group consisting of glucose oxidase, peroxidase, potassium iodide, 2,4-dichloroaniline diazonium salt, sodium nitroprusside, bromthymol blue, methyl vinyl ether, maleic anhydride, sodium hydroxide, diispropylbenzene dihydroperoxide, 3, 3', 5, 5'-tetramethylbenzidine, methyl red, tetrabromphenol blue, p-diethylamino-benzaldehyde, p-arsanilic acid, 1, 2, 3, 4-tetrahydrobenzo(h) quinolin-3-ol, derivatized pyrrole amino acid ester, bis (3',3"-diiodo-4',4"-dihydroxy-5',5"-dinitrophenyl)-3,4,5,6-tetrabromosulfonepthalein, copper sulfate, and diazonium salt, or combinations thereof.

9. A method for performing analytical reactions to determine the presence or absence of an analyte in a liquid test sample, the method comprising the steps of:
obtaining a liquid test sample from a patient and disposing the liquid test sample in a receptacle;
introducing a test strip device according to claim 1 into the receptacle;
measuring the detectable response produced to determine the presence or absence of at least one analyte present in the liquid test sample.

10. The method of claim 9, wherein the detectable response comprises a color change to the at least two analyte testing pads.
